# EUROPÄISCHE PATENTSCHRIFT

(11) **EP 1 290 145 B1**
(45) Veröffentlichungstag und Bekanntmachung des Hinweises auf die Patenterteilung: **02.11.2005**
(21) Anmeldenummer: 01953961.8
(22) Anmeldetag: 29.05.2001
(51) Int. Cl.: C12N 5/06, C12N 5/08, G01N 33/50

(54) **DREIDIMENSIONALES HAUTMODELL**
THREE-DIMENSIONAL SKIN MODEL
MODELE DE PEAU TRIDIMENSIONNEL

(30) Priorität: 31.05.2000 DE 10026789; 15.12.2000 DE 10062623
(43) Veröffentlichungstag der Anmeldung: 12.03.2003
(73) Patentinhaber: Fraunhofer-Gesellschaft zur Förderung der angewandten Forschung e.V., 80686 München (DE)
(72) Erfinder: NOLL, Michaela, 70597 Stuttgart (DE); GRAEVE, Thomas, 70597 Stuttgart (DE)
(86) Internationale Anmeldenummer: PCT/EP2001/006074
(87) Internationale Veröffentlichungsnummer: WO 2001/092477

(56) Entgegenhaltungen:
- EP-A- 0 851 227
- WO-A-91/16010
- WO-A-97/41208
- US-A- 4 485 096
- US-A- 5 478 739
- US-A- 5 945 101

## Beschreibung

Die Erfindung betrifft ein hauttypisches, dreidimensionales, vorzugsweise humanes, in vitro-Hautäquivalent, bestehend aus einem Dermisäquivalent und einem Epidermisäquivalent, und Verfahren zur Herstellung, Kultivierung und Anwendung dieses Hautäquivalents sowie dessen Bestandteile.

Hauttypische Vollhautmodelle, die auch als in vitro-Hautäquivalente bezeichnet werden, können insbesondere in der Dermatologie und in der Allergologie als Testhaut verwendet werden, um Substanzen, beispielsweise potentielle Arzneimittel oder Kosmetika, oder Agenzien, wie Licht und Wärme, auf ihre pharmakologischen Wirkungen, insbesondere Reiz-, Toxizitäts- und Entzündungswirkungen, und auf ihre Verträglichkeit zu untersuchen. Ein solches System kann darüber hinaus für vielfältige immunologische, histologische und molekularbiologische Fragestellungen eingesetzt werden. Dazu zählen zum Beispiel Studien zur Wundheilung und zur Penetration und Resorption von Substanzen. Die Untersuchungen beziehungsweise Tests von Substanzen an solchen Vollhautmodellen bieten gegenüber Tierversuchen und Versuchen mit menschlichen Probanden wesentliche Vorteile, da die damit erzielten Ergebnisse reproduzierbarer sind und die Untersuchungen kostengünstiger und schneller durchgeführt werden können.

Zur Prüfung von Rohstoffen und Produkten sind in den letzten Jahren zumeist humane Zellkulturen als in vitro-Systeme verwendet worden. Eine Weiterentwicklung der Zellkulturtechnik stellen dreidimensionale, organähnliche, humane Zellstrukturen und Cokultursysteme dar. Die damit gewonnenen Ergebnisse lassen sich noch besser auf den Menschen übertragen als die an Einzelzellkulturen gewonnenen Ergebnisse. Mit den Entwicklungen von Rheinwald und Green (Rheinwald, J. G. et al., "Serial cultivation of strains of human epidermal keratinocytes: The formation of keratinizing colonies from single cells", Cell, 6 (1975), 331-344; Green, H. et al., "Growth of cultured human epidermal cells into multiple e-pithelia suitable for grafting", Proc. Nat. Acad. Sei. USA 76 (1979), 5665-5668) hat die Kultivierung von humanen Keratinocyten und deren Anwendung in der Verbrennungsmedizin und der in vitro-Dermatologie ihre Anfänge genommen. Bislang wurden unterschiedliche Modelle rekonstruierter Haut in vitro hergestellt.

Die EP 0 197 090 B1 offenbart ein Verfahren zur Bildung eines Hautäquivalents, wobei durch Mischen einer saure Kollagenlösung mit kontraktilen Zellen, beispielsweise Fibroblasten, ein hydratisiertes Kollagengitter hergestellt wird. Nach Neutralisierung des pH-Wertes werden in dem Kollagengitter Kollagenfibrillen ausgefällt. Die kontraktilen Zellen lagern sich an das Kollagengitter an und bewirken dessen Kontraktion, wobei sich ein Dermisäquivalent bildet. Durch das Einbringen von Haut-Stanzbiopsien in das Kollagengitter können Keratinocyten aus den Stanzbiopsien auf der Oberfläche des Dermisäquivalentes wachsen, wobei sich ein Hautäquivalent bildet.

In der EP 0 285 474 B1 wird ein Hautäquivalent offenbart, das ein aus Kollagen und Fibroblasten erhaltenes Dermisäquivalent und ein mehrschichtiges Epidermisäquivalent umfaßt. Dabei wird das Dermisäquivalent mit einem menschlichen oder tierischen Explantat, beispielsweise einem Haarfollikel, beimpft, um das Epidermisäquivalent zu erhalten.

Die EP 0 020 753 B1 beschreibt ein Verfahren zur Bildung von Gewebe, insbesondere Hautgewebe, wobei ebenfalls Fibroblasten in ein hydratisiertes Kollagengitter eingebracht werden und sich nach Kontraktion des Kollagengitters ein Gewebe bildet. Auf dieses Gewebe können zuvor in vitro kultivierte Keratinocyten oder aus Vorhaut isolierte Keratinocyten aufgebracht werden, wobei sich ein Hautersatz bildet.

Aus der EP 0 418 035 B1 ist ein Gewebeäquivalent bekannt, das ein hydratisiertes Kollagengitter, welches mittels eines kontraktilen Agens wie Fibroblasten kontrahiert wird, und ein Kollagengel, welches mit einem permeablen Element in Kontakt steht, umfasst. Dabei wird das Gemisch aus Kollagen und kontraktilem Agens auf das Kollagengel aufgetragen, wobei durch den Kontakt zwischen Kollagengel und permeablem Element, beispielsweise einer Polycarbonatmembran, die radiale oder laterale Kontraktion des Kollagengitters unterbunden wird, so dass das Gitter nur bezüglich seiner Dicke kontrahiert. Nach Bildung des Dermisäquivalents können dann Keratinocyten ausgesät werden, wobei sich ein Hautäquivalent bildet.

Ferner offenbart das US-Patent Nr. 5,861,153 ein Hautäquivalent, das aus einem Epidermisäquivalent auf einem Träger besteht, wobei das Epidermisäquivalent Keratinocyten und induzierte oder nicht induzierte Vorläufer von Langerhans-Zellen umfasst. Bei dem Träger kann es sich um ein Fibroblasten enthaltendes Kollagengitter handeln oder um von der Epidermis befreite Dermisabschnitte, künstliche Membranen, einen Subkutanersatz auf der Basis von Kollagen oder synthetische Materialien.

Das US-Patent Nr. 4,963,489 beschreibt ein in vitro hergestelltes Stroma-Gewebe, wobei die Stromazellen, beispielsweise Fibroblasten, ein Grundgerüst umhüllen, das aus einem biologisch verträglichen Material, beispielsweise Cellulose, besteht. Das beschriebene System läßt sich unter anderem zur Herstellung eines dreidimensionalen Hautkultursystems verwenden, wobei Keratinocyten und Melanocyten auf dem Dermisäquivalent, das heisst der dreidimensionalen Stroma-Trägermatrix, ausgebracht werden.

Das US-Patent Nr. 5,755,814 beschreibt ein Hautmodellsystem, das sich sowohl als in vitro-Testsystem als auch für therapeutische Zwecke einsetzen lässt. Das System umfasst eine dreidimensionale vernetzte Matrix von unlöslichem Kollagen mit darin enthaltenen Fibroblasten und stratifizierte Schichten differenzierter Epidermiszellen, wobei eine Epidermiszell-Schicht in direktem Kontakt zu der Oberfläche der Kollagen-Matrix steht. Die Vernetzung der Matrix kann sowohl mittels thermischer Behandlung unter Wasserentzug als auch durch chemische Mittel, beispielsweise Carbodiimid, erfolgen.

In dem US-Patent Nr. 5,882,248 ist ein Verfahren zur Bestimmung der Wirkung chemischer Substanzen oder Agenzien auf ein menschliches Hautmodellsystem gemäß US-Patent Nr. 5,755,814 beschrieben. Die Wechselwirkung zwischen dem Hautmodellsystem und den zu testenden Substanzen wird anhand der Freisetzung von Stoffen durch Zellen des Hautmodellsystems sowie der Wirkungen auf Stoffwechsel, Proliferation, Differenzierung und Reorganisation dieser Zellen bestimmt.

In der WO 95/10600 ist darüber hinaus ein Verfahren beschrieben, mit dem ein Epidermisäquivalent gewonnen werden kann. Dieses Epidermisäquivalent kann für pharmazeutische und/oder kosmetische Sonnenbräunungs-Tests verwendet werden.

Bei den bekannten Hautmodellen wirkt sich nachteilig aus, dass diese zumeist nur aus einer oder mehreren epidermalen Schicht(en) aus Keratinocyten bestehen. In den Fällen, wo eine stratifizierte Epidermis erhalten wird, werden Gewebe-Explantate eingesetzt, die die Gefahr einer Kontamination mit Erregern in sich bergen, was bei einer späteren Verwendung des Hautäquivalents als Testhaut zu Ergebnisverfälschungen führen kann. Sofern die beschriebenen Hautmodelle einen Dermalteil besitzen, besteht dieser häufig aus spongiösem, quervernetztem Material, das neben Kollagen außerdem noch andere nicht-hauttypische Materialien enthalten kann. Sofern bei den im Stand der Technik beschriebenen Hautäquivalenten der Dermalteil nur aus Kollagen und Fibroblasten besteht, ist er einem undefinierten Schrumpfungsprozess unterworfen, der auf eine starke Schrumpfung des Kollagengels und einen Flüssigkeitsaustritt daraus zurückzuführen ist. Dies führt dazu, dass die im Stand der Technik beschriebenen Hautäquivalente sich nur in beschränktem Maße als Testhaut definierter Größe eignen und die damit erhaltenen Ergebnisse sich nur bedingt auf native menschliche Haut übertragen lassen.

Das der vorliegenden Erfindung zu Grunde liegende technische Problem besteht also darin, ein weitgehend nativer menschlicher Haut entsprechendes dreidimensionales humanes in vitro-Vollhautmodell sowie Verfahren und Mittel zu dessen Herstellung bereit zu stellen, das sowohl eine Epidermisschicht als auch eine Dermisschicht besitzt, die keinem undefinierten Schrumpfungsprozess unterworfen ist, und das sich als Testhaut definierter Größe beispielsweise zur Untersuchung pharmakologischer und kosmetischer Wirkungen einsetzen lässt.

Die Erfindung löst das ihr zu Grunde liegende Problem durch die Bereitstellung eines Verfahrens zur Differenzierung und/oder Vermehrung isolierter dermaler Fibroblasten gemäß der Ansprüche 1 bis 14, wobei die Fibroblasten in einer dreidimensionalen gelartigen Biomatrix enthaltend 3,5 bis 4,5 mg/ml Kollagen in gepuffertem serumhaltigen Zellkulturmedium, wobei die für die Herstellung der Biomatrix verwendete Kollagenlösung einen Anteil von ≥ 90 % an nicht-denaturiertem, nativen Kollagen enthält, kultiviert werden und sich dort vermehren können. Diese Biomatrix enthält neben den zu kultivierenden Fibroblasten ein aus einer Kollagenlösung konstituiertes Gerüst aus menschlichem oder tierischen Kollagen, also gewebetypische Matrixproteine. Dieses Kollagen-Fibroblasten-Gel wird erfindungsgemäß bevorzugt einer ein- bis zweitägigen Submers-Kultur unterworfen. Auf die Fibroblasten enthaltende Biomatrix werden danach Keratinocyten-Stammzellen ausgesät. In besonders bevorzugter Ausführungsform werden vorzugsweise Keratinocyten mit einem vergleichsweise hohen Anteil, beispielsweise 0,5%, 1%, 2%, 5%, 8%, oder 10% an der Keratinocyten-Zellpopulation, oder umfassend nur undifferenzierte Stammzellen, eingesetzt. Unter Verwendung spezifischer Kulturbedingungen, die insbesondere eine mehrtägige Submerskultur und eine nachfolgende mehrtägige Airlift-Kultur des Biomatrix-Systems umfassen, und spezifischer Kulturmedien durchlaufen die Keratinocyten eine Differenzierung zu einer mehrschichtigen Epidermisschicht. Erfindungsgemäß ist darüber hinaus in einer bevorzugten Ausführung vorgesehen, dass vor, während oder nach der Aussaat der Keratinocyten auch andere Zelltypen und/oder andere Zellen anderer Gewebetypen auf der Biomatrix ausgesät werden können, zum Beispiel Immunsystemzellen.

Mit Hilfe der erfindungsgemäßen Verfahren wird somit ein organoides in vitro-Hautmodell erhalten, das aus zwei gewebetypischen Schichten, nämlich einem Dermisäquivalent und einem Epidermisäquivalent, aufgebaut ist. Das organotypische Hautmodell entspricht sowohl histologisch als auch funktionell weitgehend der nativen Haut.

Durch ein spezielles Verfahren zur Kollagenextraktion und durch die Zusammensetzung der zur Bildung der Biomatrix verwendeten Kollagen-Suspension wird erreicht, dass das Dermisäquivalent im Verlauf der Kultivierungsdauer keinem undefinierten Schrumpfungsprozess unterworfen ist. Aufgrund der verwendeten Kulturverfahren und der Verwendung von Zellkulturinserts mit einer speziellen Oberflächenbeschichtung wird erreicht, dass der Dermalteil lediglich einer definierten Schrumpfung in vertikaler Richtung unterworfen wird, während eine Schrumpfung in horizontaler Richtung verhindert wird. Dadurch werden Hautäquivalente mit definiertem Durchmesser, einheitlicher Oberfläche und einem definierten Abschluss zum Rand des Kulturinserts erhalten. Durch die einheitliche Größe und einheitliche Beschaffenheit des als Testoberfläche verwendeten Vollhautmodells wird erreicht, dass bei Tests von Substanzen auf pharmakologische und/oder kosmetische Effekte die Qualität der Ergebnisse gesteigert und die Testergebnisse reproduzierbarer werden.

Eine besonders bevorzugte Ausführungsform der Erfindung umfasst die Kultivierung dermaler Fibroblasten in einer dreidimensionalen gelartigen Biomatrix gebildet gemäß der Ansprüche 1 bis 14 zur Vermehrung der Fibroblasten oder zur Herstellung eines Dermisäquivalentes und/oder eines Hautäquivalentes.

Im Zusammenhang mit der vorliegenden Erfindung bedeutet der Begriff "Kultivieren von Zellen" ein, vorzugsweise in vitro stattfindendes, Aufrechterhalten der Lebensfunktionen von Zellen, beispielsweise Fibroblasten, in einer geeigneten Umgebung, beispielsweise unter Zu- und Abfuhr von Stoffwechseledukten und -produkten, insbesondere auch eine Vermehrung der Zellen.

Im Zusammenhang mit der vorliegenden Erfindung werden unter dermalen Fibroblasten natürlicherweise vorkommende, insbesondere in der Dermis vorkommende Fibroblasten oder gentechnisch veränderte Fibroblasten oder deren Vorläufer verstanden. Fibroblasten stellen die Vorläufer dermaler Fibrocyten, das heisst spindelförmiger Zellen des Haut-Bindegewebes mit ovalem Kern und langen Fortsätzen, dar. Die Fibroblasten können tierischer oder menschlicher Herkunft sein.

Die zur Kultivierung der Fibroblasten vorgesehene Biomatrix enthält also die zu kultivierenden Fibroblasten und ein aus einer, vorzugsweise frischen, Kollagenlösung menschlichen oder tierischen Ursprungs neu konstituiertes Kollagengerüst einer Konzentration von mindestens 3 mg Kollagen pro ml Biomatrix, erfindungsgemäß 3,5 bis 4,5 mg Kollagen pro ml Biomatrix. Das Kollagengerüst wird aus einer, vorzugsweise zellfreien, sauren Lösung von Kollagen I, gewonnen, wobei die Proteinkonzentration der Kollagenlösung vorzugsweise 5 bis 7 mg/ml beträgt. Der pH-Wert der Kollagenlösung beträgt 0,1 bis 6,9, vorzugsweise 2,0 bis 5,0, bevorzugt 3,0 bis 4,5, insbesondere 3,2 bis 4,2 und besonders bevorzugt 3,8. Zur Herstellung der erfindungsgemäßen Fibroblasten-haltigen Biomatrix wird die Kollagenlösung bei 2°C bis 10°C, vorzugsweise bei 4°C, mit einer Lösung, enthaltend ein, vorzugsweise fünffach konzentriertes, Zellkulturmedium, vorzugsweise fünffach konzentriertes M199-Zellkulturmedium, Puffer, vorzugsweise Hepes-Puffer, Serum, vorzugsweise fötales Kälberserum (FCS), und Chondroitin-(4/6)-sulfat, und vorzugsweise 1,5 x 10⁵/ml Fibroblasten, insbesondere vorkultivierten Fibroblasten, versetzt und gut gemischt. Dieses Gemisch wird in die Vertiefungen einer Mikrokulturplatte mit 24 Vertiefungen, wobei jede Vertiefung einen Durchmesser von 10 mm aufweist, gegeben und durch Erhöhung der Temperatur auf beispielsweise Raumtemperatur oder 37°erfolgt eine Gelierung. Nach dem Gelieren der Fibroblasten-Kollagengele wird Fibronectin, vorzugsweise humanes Fibronectin, auf die Gele gegeben. Bei Fibronectinen handelt es sich um in Fibroblasten produzierte Struktur- beziehungsweise Adhäsionsproteine, deren Funktion in vivo in der Bindung an andere Makromoleküle, beispielsweise Kollagen, und in der Anheftung von Zellen an Nachbarzellen besteht. Durch die Zugabe von Fibronektinen zur Fibroblasten-Kollagenmatrix wird also die Bindung der Fibroblasten sowohl an Kollagen als auch untereinander begünstigt. Die anschließende Kultivierung der Fibroblasten im Kollagengel erfolgt vorzugsweise in Submers-Kultur. Im Zusammenhang mit der vorliegenden Erfindung wird unter einer "Submers-Kultivierung" oder einer "Submers-Kultur" ein Verfahren zur Kultivierung von Zellen verstanden, wobei die Zellen mit einer Nährlösung bedeckt sind. Die Fibroblasten enthaltende Biomatrix wird also mit Zellkulturmedium überschichtet und bei 37°C inkubiert.

In einer vorteilhaften Ausführungsform der Erfindung können die in der Biomatrix kultivierten Fibroblasten wieder aus der Biomatrix herausgelöst und gegebenenfalls erneut in eine Biomatrix eingebracht werden, wobei die Zellen nach Herauslösen ihre spezifischen Stoffwechselleistungen und ihren Differenzierungsstatus nicht verlieren. Das erfindungsgemäße Verfahren gestattet es also, eine Zwischenkultivierung der Fibroblasten in der Biomatrix durchzuführen. Bei einer geringen Ausgangsmenge an Fibroblasten bietet das erfindungsgemäße Verfahren daher den Vorteil, dass genügend Zellmaterial für die Herstellung von Dermisäquivalenten und/oder Hautäquivalenten zur Verfügung gestellt werden kann.

Eine weitere vorteilhafte Ausgestaltung der Erfindung sieht vor, das dermale Fibroblasten, die in ihrer Funktion, ihrer Morphologie und/oder ihrem Differenzierungsstatus überprüft werden sollen, in eine vorstehend genannte dreidimensionale Biomatrix eingebracht, kultiviert und dabei und/oder danach überprüft werden. Die Erfindung betrifft daher auch unter Verwendung dermaler Fibroblasten durchgeführte Screening- und Diagnoseverfahren, wobei die Fibroblasten gemäß dem vorstehend beschriebenen Verfahren kultiviert und dabei und/oder anschließend untersucht werden können, zum Beispiel auf pharmakologische, toxikologische, physiologische, morphologische und/oder molekularbiologische Parameter.

In einer weiteren vorteilhaften Ausführungsform der Erfindung werden die dermalen Fibroblasten in der dreidimensionalen Biomatrix, wie vorstehend beschrieben, so kultiviert, dass anschließend ein Dermisäquivalent gewonnen werden kann. Im Zusammenhang mit der vorliegenden Erfindung wird unter einem "Dermisäquivalent" eine Bindegewebe-artige Schicht aus Kollagen und Fibroblasten verstanden, die weitgehend der nativen Dermis entspricht.

Das so erhaltene Dermisäquivalent kann für Screening- und Diagnoseverfahren verwendet werden, insbesondere zur Untersuchung der Wirkungen chemischer Substanzen, beispielsweise potentieller Arzneimittel oder Bestandteile von Kosmetika, oder anderer Agenzien. Im Zusammenhang mit der vorliegenden Erfindung werden unter dem Begriff "Agens" oder "Agenzien" insbesondere auf die Haut oder Hautzellen wirkende physikalische Mittel wie Licht, Wärme, oder ähnliches, verstanden. Die Erfindung betrifft daher auch Screening- und Diagnoseverfahren unter Verwendung der erfindungsgemäß hergestellten Dermisäquivalente.

Eine bevorzugte Ausführung der Erfindung umfasst die Behandlung des Dermisäquivalents in An- und Abwesenheit der zu untersuchenden Substanz und/oder des zu untersuchenden Agens und den Vergleich der beobachteten Auswirkungen auf die Zellen oder Zellbestandteile des Dermisäquivalents.

Eine weitere bevorzugte Ausführung der Erfindung umfasst ein Verfahren zur Untersuchung der Penetration von Substanzen unter Verwendung des erfindungsgemäß hergestellten Dermisäquivalents und unter Verwendung eines erfindungsgemäßen Hautäquivalents, das aus einem Dermisäquivalent und einem Epidermisäquivalent besteht.

Eine besonders bevorzugte Ausführungsform der Erfindung betrifft auch ein vorgenanntes Verfahren zur Kultivierung dermaler Fibroblasten in einer Biomatrix zur Herstellung eines aus Dermisäquivalent und Epidermisäquivalent bestehenden Hautäquivalentes. Dabei werden ein bis drei Tage, vorzugsweise zwei Tage, nach der vorstehend beschriebenen Herstellung und Inkubation der Kollagen-Fibroblasten-Gele Keratinocyten auf das Gel ausgesät.

Im Zusammenhang mit der vorliegenden Erfindung werden unter "Keratinocyten" Zellen der Epidermis, die verhornendes Plattenepithel bilden, oder gentechnisch veränderte Keratinocyten oder deren Vorläufer verstanden, die tierischer oder menschlicher Herkunft sein können. Da die Ausbildung einer gut differenzierten Epidermis mit intakter Verhornung in starkem Maße vom Anteil basaler Stammzellen in den verwendeten Keratinocyten abhängt, handelt es sich bei den auf das Kollagengel ausgesäten Keratinocyten vorzugsweise um möglichst undifferenzierte Keratinocyten-Stammzellen aus humanem Biopsiegewebe, das heisst Cytokeratin 19- beziehungsweise Integrin β1-positive basale Stammzellen. Vorzugsweise handelt es sich dabei um vorkultivierte Zellen, besonders bevorzugt um Keratinocyten in der ersten oder in der zweiten Zellpassage. Die Aussaat der Keratinocyten auf die Biomatrix erfolgt vorzugsweise in einem Zellkulturmedium, besonders bevorzugt in KBM-Medium (Clonetics), das 5% fötales Kälberserum enthält. Anschließend wird die Biomatrix mit KBM-Medium, enthaltend humanen epidermalen Wachstumsfaktor (hEGF) (0,1 µg/500 ml Medium), BPE (15 mg/500 ml Medium) und 0,8 mM CaCl₂, überschichtet und einer vorzugsweise 1 - bis 3-tägigen Sübmers-Kultivierung unterworfen. Eine vollständige Differenzierung der Keratinocytenschichten wird durch eine Airlift-Kultur mit 1,8 mM CaCl₂ enthaltendem KBM-Medium ohne hEGF und BPE erreicht. Im Zusammenhang mit der vorliegenden Erfindung wird unter einer "Airlift-Kultur" eine Kultur verstanden, wobei die Höhe des Nährmedienspiegels genau auf die Höhe der Biomatrix abgestimmt ist, während die Keratinocyten oder durch die Keratinocyten gebildeten Zellschichten über dem Nährmedienspiegel liegen und vom Nährmedium nicht bedeckt werden, das heisst die Kultivierung erfolgt an der Grenzschicht Luft-Nährmedium, wobei die Versorgung der Kulturen von unten her erfolgt. Dazu werden die Inserts aus der Mikrotiterplatte mit 24 Vertiefungen in die Vertiefungen einer Mikrotiterplatte mit 6 Vertiefungen mit jeweils einem Durchmesser von 3,5 cm übertragen. Nach einer, vorzugsweise 12- bis 14-tägigen Airlift-Kultur entwickelt sich ein hauttypisches, aus Dermisäquivalent und Epidermisäquivalent bestehendes in vitro-Vollhautmodell.

Das erfindungsgemäße Verfahren zur Herstellung eines in vitro-Vollhautmodells kann vorteilhafterweise so modifiziert werden, dass vor, während oder nach der Aussaat von Keratinocyten weitere Hautzelltypen, wie Melanocyten, Immunzellen und/oder Endothelzellen, auf der Biomatrix ausgesät und weiter kultiviert werden können.

Die Erfindung betrifft daher auch ein hauttypisches in vitro-Vollhautmodell, insbesondere ein humanes in vitro-Vollhautmodell, das nach dem erfindungsgemäßen Verfahren und einem gegebenenfalls anschließenden und/oder vorausgehenden Kultivierungsverfahren herkömmlicher Art hergestellt wurde und das mindestens 2 bis 4 proliferative, einige differenzierende und mindestens 4 bis 5 verhornte Zellschichten umfasst, wobei das Epidermisäquivalent Stratum basale, Stratum spinosum, Stratum granulosum und Stratum corneum umfasst und wobei zwischen dem Dermisäquivalent und dem Epidermisäquivalent eine funktionsfähige Basalmembran aus Matrixproteinen enthalten ist und wobei darüber hinaus hauttypische Proteine wie Fillgrin, Ki-67 und Cytokeratin 10 exprimiert werden.

Aufgrund der Komplexität des hergestellten Hautmodells kann dieses gezielt für verschiedene Fragestellungen der chemisch-pharmazeutischen Industrie und der kosmetischen Industrie verwendet werden. Insbesondere eignet sich das erfindungsgemäß hergestellte Hautäquivalent zur Produktprüfung, beispielsweise im Hinblick auf Wirksamkeit, unerwünschte Nebenwirkungen, beispielsweise Reiz-, Toxizitäts- und Entzündungswirkungen oder allergieauslösende Wirkungen, oder Verträglichkeit von Substanzen. Dabei kann es sich um Substanzen handeln, die potentielle Verwendung als Arzneimittel, insbesondere als Dermatika, finden sollen, oder um Substanzen, die Bestandteil von Kosmetika sind. Das erfindungsgemäß hergestellte Hautäquivalent kann beispielsweise auch für Studien zur Resorption, zum Transport und/oder zur Penetration von Substanzen verwendet werden. Darüber hinaus eignet es sich auch zur Untersuchung anderer Agenzien, wie Licht oder Wärme, beispielsweise zur Untersuchung der Phototoxizität, also der schädigenden Wirkung von Licht unterschiedlicher Wellenlänge, auf Zellstrukturen. Das erfindungsgemäß hergestellte Hautäquivalent kann selbstverständlich auch zur Untersuchung der Wundheilung eingesetzt werden.

Die Wirkungen von Substanzen oder Agenzien auf menschliche Haut lassen sich beispielsweise anhand der Freisetzung von Stoffen, beispielsweise Cytokinen oder Mediatoren, durch Zellen des humanen Hautmodellsystems, sowie der Wirkungen auf Genexpression, Stoffwechsel, Proliferation, Differenzierung und Reorganisation dieser Zellen ermitteln. Unter Verwendung von Verfahren zur Quantifizierung der Zellschädigung, insbesondere unter Verwendung eines Vitalfarbstoffes, wie eines Tetrazoliumderivates, können beispielsweise cytotoxische Wirkungen auf Hautzellen nachgewiesen werden. Die Tests von Substanzen oder Agenzien an dem erfindungsgemäßen humanen Hautäquivalent können sowohl histologische Verfahren als auch immunologische und/oder molekularbiologische Verfahren umfassen.

Eine bevorzugte Ausführungsform der Erfindung umfasst daher Verfahren zur Untersuchung der Wirkung, insbesondere der pharmakologischen Wirkungen, von Substanzen oder Agenzien auf menschliche Haut unter Verwendung des erfindungsgemäß hergestellten humanen Hautäquivalentes. In einer besonders bevorzugten Ausführungsform wird dabei ein EZ4U-Test durchgeführt. EZ4U ist ein nicht-toxisches wasserlösliches gelbes Tetrazoliumsalz, das von lebenden Zellen zu intensiv gefärbten Formazanen reduziert werden kann. Die Reduktion erfordert intakte Mitochondrien und der Test kann daher zum Nachweis der Vitalität von Zellen eingesetzt werden.

Eine weitere bevorzugte Ausführungsform der Erfindung umfasst ein Verfahren zur Untersuchung der Penetration von Substanzen, wobei sowohl ein erfindungsgemäß hergestelltes Dermisäquivalent als auch ein erfindungsgemäß hergestelltes Hautäquivalent mit den zu untersuchenden Substanzen behandelt werden und die bei beiden Systemen erhaltenen Ergebnisse miteinander verglichen werden.

In einer besonders bevorzugten Ausführungsform der Erfindung werden die Wirkungen chemischer Substanzen oder anderer Agenzien auf spezielle Hauttypen untersucht. Dabei werden Zellen definierter Hauttypen, beispielsweise Hauttypen mit wenig Pigmenten und/oder Hauttypen mit vielen Pigmenten, zur Etablierung erfindungsgemäßer Hautäquivalente eingesetzt und diese werden im Hinblick auf die Wirkung von Substanzen oder Agenzien getestet.

In einer weiteren besonders bevorzugten Ausführungsform der Erfindung wird das erfindungsgemäß hergestellte Hautäquivalent als Modellsystem zu Untersuchungen von Hautkrankheiten und zur Entwicklung neuer Behandlungsmöglichkeiten für Hautleiden verwendet. Beispielsweise können Zelllinien von Patienten mit einer bestimmten Hautkrankheit verwendet werden, um daraus patientenspezifische Hautmodellsysteme zu etablieren und daran die Wirksamkeit bestimmter Therapien und/oder Medikamente zu untersuchen und zu beurteilen.

Die Erfindung betrifft auch eine, vorzugsweise gelartige, Biomatrix, in der die vorgenannten Kultivierungsverfahren durchgeführt werden können, und zwar eine Biomatrix mit dermalen Fibroblasten. Die erfindungsgemäß vorgesehene Kombination aus Biomatrix und darin kultivierten dermalen Fibroblasten kann, wie vorstehend beschrieben, zur Herstellung eines Dermisäquivalentes und/oder eines organoiden Vollhautmodells verwendet werden.

Unter einer Biomatrix wird eine Gelstruktur verstanden, die Kollagen, Zellkulturmedium, Serum und Puffer, beispielsweise Hepes-Puffer, enthält. Die Kollagenlösung, die für die Herstellung der Biomatrix verwendet wird, ist eine Lösung, die einen hohen Anteil an nicht denaturiertem, nativem Kollagen in saurem, wässrigem Medium enthält, vorzugsweise mit einem pH-Wert von 3,8, beispielsweise in Essigsäure, bevorzugt in 0,1 %iger Essigsäurelösung. Ein hoher Anteil von nicht denaturiertem Kollagen bedeutet einen Anteil am Gesamtkollagen in Lösung von ≥ 50%, insbesondere ≥ 60%, ≥ 70%, ≥ 80%, erfindungsgemäß ≥ 90% oder ≥ 95 %, vorzugsweise ≥ 99%. In einer bevorzugten Ausführungsform wird dabei kein lyophilisiertes Kollagen verwendet. Der Kollagengehalt der Lösung beträgt vorteilhafterweise 3 mg Kollagen pro ml Lösung bis 8 mg Kollagen pro ml Lösung, bevorzugter 5 mg Kollagen pro ml Lösung bis 7 mg Kollagen pro ml Lösung, am bevorzugtesten 6 mg Kollagen pro ml Lösung. Vorzugsweise wird dabei Kollagen verwendet, das nach Isolierung, beispielsweise aus Rattenschwänzen, in 0,1 %iger Essigsäure drei bis vierzehn Tage bei 4°C unter Rühren inkubiert wurde und wobei nicht gelöste Kollagenanteile abzentrifugiert wurden. Bevorzugte Zellkulturmedien sind DMEM (Dulbecco's Modified Eagle Medium) und M199. Jedoch kann auch jedes andere beliebige Zellkulturmedium verwendet werden, welches die Kultivierung von Fibroblasten ermöglicht. Als Serum wird vorzugsweise fötales Kälberserum (FCS) verwendet und als Puffer zum Beispiel Hepes-Puffer. Der pH-Wert der Lösung aus Zellkulturmedium, Puffer und Serum beträgt in bevorzugter Ausführung 7,5 bis 8,5, beispielsweise 7,6 bis 8,2, insbesondere 7,8. Selbstverständlich kann die Biomatrix weitere Faktoren, beispielsweise Wachstumsfaktoren, Adhäsionsmittel, Antibiotika, Selektionsmittel und ähnliche enthalten.

Die Erfindung betrifft daher auch Verfahren zur Herstellung einer dermale Fibroblasten enthaltenden Biomatrix, wobei in einem ersten Schritt frisches Kollagen menschlichen oder tierischen Ursprungs, beispielsweise aus Rattenschwänzen, hergestellt wird, indem aus kollagenhaltigem Gewebe isolierte Kollagenfasern in Pufferlösung gesammelt, in Alkohol oberflächlich desinfiziert und anschließend in Pufferlösung gewaschen und anschließend in eine saure Lösung eines pH-Wertes von 0,1 bis 6,9, vorzugsweise 2,0 bis 5,0, besonders bevorzugt 3,0 bis 4,0, insbesondere 3,3, zum Beispiel eine 0,1 %ige Essigsäurelösung, überführt werden. Anschließend wird in einem weiteren Schritt das in der Lösung befindliche Kollagen bei 2 bis 10°C, insbesondere 4°C, für einige Tage, zum Beispiel 3 bis 14 Tage, gerührt, die nicht gelösten Kollagenanteile werden abzentrifugiert und eine Kollagenlösung mit einem Kollagengehalt von 3 mg/ml bis 8 mg/ml bei 2 bis 10°C, zum Beispiel 4°C, aufbewahrt. Selbstverständlich ist es möglich, die Lösung in gefrorenem Zustand zwischenzulagern, zum Beispiel bei -10°C bis -80°C, insbesondere -20°C. Zur Herstellung der erfindungsgemäßen Fibroblasten-haltigen Biomatrix wird in einem dritten Schritt die Lösung aus, vorzugsweise fünffach konzentriertem, Zellkulturmedium, Serum und Puffer mit vorkultivierten und abzentrifugierten Fibroblasten gemischt, wobei vorzugsweise 1 x 10⁵ bis 2 x 10⁵ Zellen pro ml, bevorzugt 1,5 x 10⁵ Zellen pro ml, verwendet werden. Diese Lösung beziehungsweise Suspension eines pH-Wertes von 7,5 bis 8,5, bevorzugt 7,6 bis 8,2, insbesondere 7,8, wird anschließend, besonders bevorzugt im Verhältnis 1:2, mit der vorgenannten Kollagenlösung bei 2 bis 10°C, insbesondere 4°C, gemischt. Anschließend wird die Gellösung in Kulturgefäße pipettiert und nach Gelieren bei 37°C mit Medium überschichtet. Sodann wird die Biomatrix mindestens 2 Tage kultiviert und anschließend können Keratinocyten darauf ausgebracht werden.

Weitere vorteilhafte Ausgestaltungen der Erfindung ergeben sich aus der Beschreibung.

Die Erfindung wird anhand der folgenden Figuren und Beispiele näher erläutert.
Figur 1 zeigt einen Längsschnitt nativer menschlicher Haut und einen Längsschnitt eines erfindungsgemäß hergestellten humanen Hautäquivalents.
Figur 2 stellt ein Säulendiagramm dar, das die prozentuale Veränderung des Zellmetabolismus von Hautäquivalenten zeigt, die im EZ4U-Test 48 Stunden mit zu testenden Substanzen inkubiert wurden.
Figur 3 stellt in grafischer Form die Veränderung des Interleukin 1 α-Gehaltes in den Medienüberständen von Hautäquivalenten dar, die 24 Stunden (24h-1) und 48 Stunden (24h-2) mit unterschiedlichen SDS-Konzentrationen inkubiert worden waren.
Figur 4 zeigt die Veränderung des Interleukin 1 α-Gehaltes in den Medienüberständen von Hautäquivalenten, die 24 Stunden (24h-1) und 48 Stunden (24h-2) mit zu testenden Substanzen inkubiert worden waren.
Figur 5 zeigt die Veränderung des PGE₂-Gehaltes in den Medienüberständen von Hautäquivalenten dar, die 24 Stunden (24h-1) und 48 Stunden (24h-2) mit unterschiedlichen SDS-Konzentrationen inkubiert worden waren.
Figur 6 zeigt in grafischer Form die Veränderung des PGE₂-Gehaltes in den Medienüberständen von Hautäquivalenten, die 24 Stunden (24h-1) und 48 Stunden (24h-2) mit zu testenden Substanzen inkubiert worden waren.

### Beispiel 1:

### Herstellung eines dreidimensionalen humanen Hautäquivalents

### Herstellung einer Gellösung

20 Teile fünffach konzentriertes M 199-Zellkulturmedium (Life Technologies), 10 Teile HEPES-Puffer (4,76 g in 100 ml PBS-Lösung, pH-Wert 7,3) und 1 Teil Chondroitin-(4,6)-sulfat (5 mg/ml in PBS) werden gemischt und der pH-Wert des Gemisches wird auf 7,8 eingestellt. Das Gemisch wird sterilfiltriert und anschließend mit 10 Teilen fötalem Kälberserum versetzt.

### Herstellung einer Kollagenlösung

Zur Herstellung einer Kollagenlösung wird kollagenhaltiges Gewebe, wie zum Beispiel Sehnen aus Rattenschwänzen, verwendet. Alle Arbeiten werden unter sterilen Bedingungen mit sterilen Materialien durchgeführt. Die Rattenschwänze werden nach Lagerung bei -20°C mit 70%-igem Alkohol oberflächlich desinfiziert. Die Haut der Rattenschwänze wird abgezogen und die einzelnen Kollagenfasern werden herausgezogen. Bei Verwendung anderer Ausgangsgewebe können gegebenenfalls vorhandene Zellen schonend durch mechanische, enzymatische oder chemische Behandlung entfernt werden. Die Kollagenfasern werden in phosphatgepufferter Kochsalzlösung (PBS) (pH 7,2) gesammelt, in 70%-igem Alkohol 10 min oberflächlich desinfiziert und anschließend gründlich mit PBS gewaschen. Das Gewicht der Fasern wird bestimmt und die Fasern werden in eine 0,1%-ige essigsäurelösung überführt (Endkonzentration etwa 8 bis 12 mg/ml). Dieser Ansatz wird etwa 3 bis 14 Tage bei 4°C gerührt und anschließend werden die nicht gelösten Kollagenteile mittels Zentrifugation (1000 Upm, 1 Stunde, 8°C) entfernt. Dadurch liegt das Kollagen in Lösung und nicht in faser-, Gerüst- oder Matrixform vor.

### Herstellung der dermale Fibroblasten enthaltenden Kollagengele (Ansatz für 24 Inserts)

16 ml Kollagenlösung werden in ein 50 ml-Zentrifugenröhrchen gegeben und auf Eis gestellt. Vorkultivierte dermale Fibroblasten werden geernet und ausgezählt. 1,2 x 10⁶ Fibroblasten werden in 8 ml eiskalte Gellösung aufgenommen, gut suspendiert und luftblasenfrei in die Kollagenlösung gegeben. Kollagenlösung. Gellösung und Fibroblasten werden gut gemischt. Jeweils 600 µl des Gemisches werden vorsichtig in die Vertiefung einer Mikrotiterplatte mit 24 Vertiefungen (Durchmesser je Vertiefung 10 mm) gegossen. Durch eine zweiminütige Inkubation bei 37°C erfolgt eine Gelierung des Gemisches. Nach dem Gelieren des Gemisches werden jeweils 50 µl Fibronectin (5 µg/ml) auf jedes Insert gegeben. Nach einer 10-minütigen Inkubation bei 37°C beziehungsweise einer 30-minütigen Inkubation bei Raumtemperatur wird pro Vertiefung 1 ml M199-Medium zugegeben, wobei die Inserts mit dem Medium überschichtet werden. Die im Gel enthaltenen Fibroblasten werden etwa 1 bis 2 Tage dieser Submers-Kultivierung bei 37°C unterworfen, wobei jeweils nach 12 Stunden das Medium gegen frisches Medium ausgetauscht wird.

### Aussaat der Keratinocyten und Kultivierung der Hautäquivalente

Vor der Aussaat der Keratinocyten wird zunächst vorsichtig das Medium in den Vertiefungen der Mikrotiterplatte und von den Gelen abgesaugt. Dann werden pro Vertiefung 500 µl KBM-Medium (Clonetics), enthaltend 5% FCS. Die Gele werden mit jeweils 50 µl Fibronectin-Lösung beschichtet und 1 Stunde bei 37°C inkubiert. Dann werden pro Gel 100.000 Keratinocyten in 50-100 µl KBM-Medium, enthaltend 5% FCS, ausgesät und 1 bis 2 Stunden bei 37°C inkubiert. Anschließend werden 500 µl KBM-Medium, enthaltend 5% FCS, 8 mM CaCl₂, hEGF (0,1 µg/500 ml Medium) und BPE (15 mg/500 ml Medium), zugegeben und die Gele werden 1 bis 3 Tage einer Submers-Kultivierung unterworfen, wobei das Medium täglich gegen frisches Medium ausgetauscht wird. Danach werden die Gele weitere 2 bis 3 Tage in jeweils 1 bis 1,5 ml KBM-Medium, enthaltend 2% FCS, 8 mM CaCl₂, hEGF und BPE einer Submers-Kultivierung unterworfen. Danach werden die Gele mit dem sich entwickelnden Hautäquivalent einer Airlift-Kultivierung unterworfen. Dazu werden die Gele in eine Platte mit 6 Vertiefungen umgesetzt und pro Vertiefung werden 1,5 bis 2 ml KBM-Medium mit einem CaCl₂-Gehalt von 1,88 mM ohne hEGF und BPE zugegeben, wobei der Spiegel des Mediums genau auf die Höhe des Gels abgestimmt wird, während die Keratinocyten oder die durch Keratinocyten gebildeten Schichten nicht vom Medium bedeckt werden. Die Airlift-Kultivierung wird mindestens 12 bis 14 Tage fortgeführt.

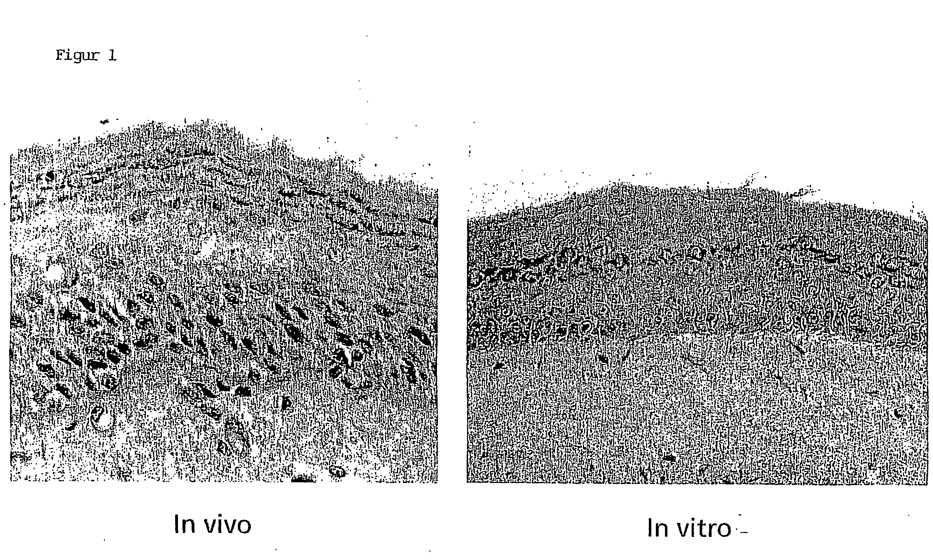

Die Figur 1 stellt vergleichend native menschliche Haut und ein erfindungsgemäßes humanes Hautäquivalent dar.

### Beispiel 2:

### Test chemischer Substanzen an einem dreidimensionalen humanen Hautäquivalent

Substanzproben wurden zur Testung am humanen Hautäquivalent eingesetzt. Eine möglicherweise irritative Wirkung der Proben auf das Vollhautmodell sollte nach 48 Stunden Inkubationszeit über den EZ4U-Metabolismus geprüft werden. Die Sekretion von Il1α und PGE₂ sollte in den Medienüberständen nach 24 Stunden und nach 48 Stunden mittels ELISA ermittelt werden. Als Referenzsubstanz wurden unterschiedliche Konzentrationen von SDS mitgeführt. Zum Abschluss wurden alle getesteten Hautäquivalente fixiert und der morphologische Aufbau an gefärbten Paraffinschnitten geprüft und ausgewertet.

Bei dem als Referenzsubstanz verwendeten SDS wurden die Expositionszeit (Et50) von 1 %igem SDS und die Ec50-Konzentrationen über 24 Stunden beziehungsweise 48 Stunden Inkubationszeit ermittelt.

Die Hautäquivalente wurden je in einem 6-well mit 1 ml Medium angesetzt (KGM ohne hEGF ohne BPE, und mit 1,8 mM CaCl₂). Die Inkubation der Proben auf der Oberfläche der entsprechenden Hautmodelle erfolgte nach folgendem Schema:
Tag 1: Aufbringen von 3 µl Substanz morgens und nachmittags.
Tag 2: Medienwechsel und Einfrieren der Medienüberstände für die Il1α- und PGE₂-Bestimmung. Behandlung mit 3 µl Substanz morgens und nachmittags.
Tag 3: Einfrieren der Medienüberstände für die Illα- und PGE₂-Bestimmung. Ermittlung des Zellmetabolismus im EZ4U-Test über einen Zeitraum von 2 Stunden. Fixierung der Präparate und Anfertigung von gefärbten Paraffinschnitten für die histologische Auswertung.

Die mitgeführten Negativkontrollen wurden entsprechend mit 3 µl PBS behandelt. Als Referenzsubstanz beziehungsweise Positivkontrolle wurde SDS in unterschiedlichen Konzentrationen (0,01%, 0,05%, 0,1%, 0,5%, 1%) verwendet. Von allen Proben wurden Doppelwerte angesetzt.

Der EZ4U-Test wurde wie folgt durchgeführt: Nach 48-stündiger Inkubation wurde der Zellmetabolismus der einzelnen Hautäquivalente anhand eines Vitalfarbstoffes (Tetrazoliumderivat) photometrisch ermittelt. Alle Äquivalente wurden vor der Durchführung es EZ4U-Tests dreimal vorsichtig mit 1,5 ml PBS gewaschen. Eine Umsatzkinetik der Negativkontrollen, Positivkontrollen und der mit den Proben behandelten Äquivalente wurde bei 450 nm (mit 620 nm als Referenzwellenlänge) über einen Zeitraum von 2 Stunden erstellt. Hierfür wurden, wie bereits beschrieben, 750 ml Assay-Medium und 75 µl Farbstoff pro Insert bei 37°C inkubiert. Für SDS wurde sowohl die Ec50 als auch die Et50 bestimmt. Die Ermittlung der Et50 erfolgte nach unterschiedlichen Expositionszeiten von 1% SDS (3 sec, 30 sec, 60 sec, 5 min, 15 min).

Der Gehalt an I11α und PGE₂ in den Medienüberständen nach 24 Stunden und 48 Stunden Inkubation der Proben wurde anhand kommerziell erhältlicher ELISA Testkits nach Vorschrift bestimmt.

Die Hautäquivalente wurden histologisch untersucht, wobei die Präparate in Bouin's Solution fixiert und in Paraffin eingebettet, histologische Schnitte angefertigt und gefärbt wurden.

Die Ermittlung des Zellmetabolismus von Negativkontrolle, Referenz beziehungsweise Positivkontrolle und der Probenäquivalente erfolgte über die Berechnung der Absorptionsdifferenz (ΔOD) Die Veränderung der ΔOD durch den Referenzstandard in unterschiedlichen Konzentrationen und durch 48-stündige Inkubation der Proben wurde bezogen auf die unbehandelte Kontrolle (Negativkontrolle, 100%) in Prozent umgerechnet.

Aus den ermittelten Werten wurde für SDS eine Dosis-Wirkungskurve beziehungsweise eine Zeit-Wirkungskurve erstellt und die Expositionszeit (Et50) beziehungsweise Konzentration (Ec50) ermittelt, die eine 50%ige Zellschadigung hervorruft. Da die Proben lediglich unverdünnt und alle über einen Zeitraum von 48 Stunden inkubiert wurden, wurde die jeweils ermittelte ΔOD prozentual zur Kontrolle dargestellt.

### Ergebnisse

### a) Cytotoxizität der Proben

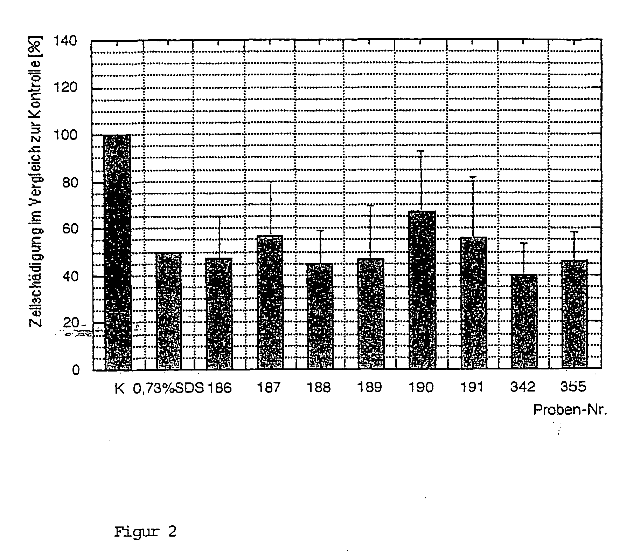

Der Zellmetabolismus der Proben wurde nach 48 Stunden Inkubation photometrisch bestimmt. Anhand der EZ4U-Umsatzkinetiken wurde die jeweilige Veränderung des Zellmetabolismus ermittelt, prozentual zur unbehandelten Kontrolle berechnet und mit der mitgeführten Referenzsubstanz verglichen. In Tabelle 1 sind die Ergebnisse dieses Testes dargestellt. Figur 2 zeigt die Veränderungen des Zellmetabolismus von Hautäquivalenten nach 48-stündiger Inkubation von Hautäquivalenten mit unterschiedlichen Konzentrationen der Referenzsubstanz SDS.

**Tabelle 1:**

| Prozentuale Veränderung des Zellmetabo-lismus nach 48 Stunden Inkubation mit den Proben, relativ zur unbehandelten Kontrolle (100%) | |
|---|---|
| **Proben-Nr.** | **AST-2000** |
| 0 (Kontrolle) | 100% |
| SDS 0,73% | 50% |
| 186 | 47,6±17,3% |
| 187 | 56,6±23,4% |
| 188 | 45±13,8% |
| 189 | 46,5±22,8% |
| 190 | 67±25,6% |
| 191 | 55,7±25,9% |
| 342 | 39,7±13,6% |
| 355 | 46,1±12,5% |

### b) Cytokinausschüttung am Beispiel von Interleukin 1a (II1a)

Die induzierte Sekretion des Cytokins I11α wurde nach einmal 24 Stunden und nach einer anschließenden weiteren Inkubation für 24 Stunden mit Testsubstanzen in den Medienüberständen der Äquivalente durch ELISA quantifiziert. Als Testsubstanzen wurden die Proben sowie unterschiedliche Konzentrationen von SDS verwendet.

### Sekretion von I11α nach SDS Stimulation

Die Ausschüttung von I11α steigt im Haut-Modell mit zunehmender SDS-Konzentration kontinuierlich an und erreicht ein Maximum bei 1 % SDS von mehr als 100 pg/ml pro Hautäquivalent. Die Werte sind nach der zweiten Inkubation insgesamt noch erhöht.

### Sekretion von I11α nach Inkubation mit den Proben

Die I11α-Ausschüttung durch die Proben war im Haut-Modell nach 24 Stunden Inkubation relativ gering (15-25 pg/ml), nach einer zweiten Inkubation von 24 Stunden konnten jedoch deutlich erhöhte II1α Werte gemessen werden.

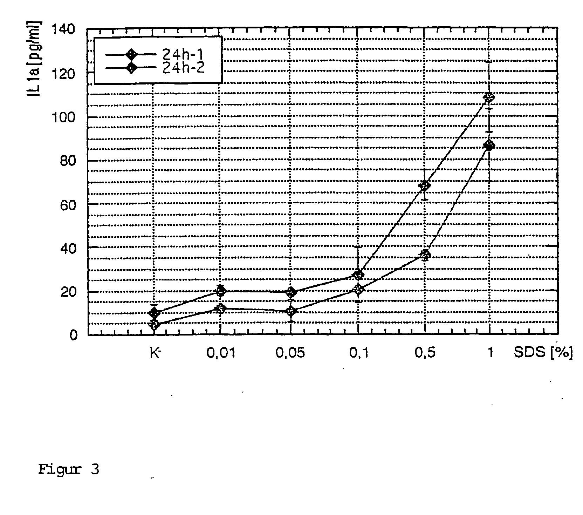

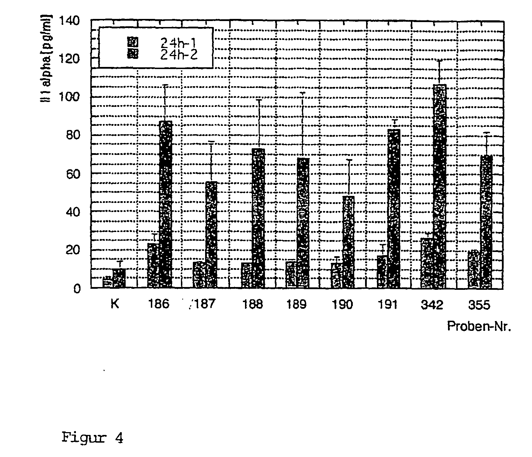

In Figur 3 sind die mit der Referenzsubstanz SDS erhaltenen Ergebnisse gezeigt. Figur 4 zeigt die mit den getesteten Proben erhaltenen Ergebnisse.

### c) Expression des Eicosanoids Prostaglandin E₂(PGE₂)

Die Synthese des Entzündungsmediators PGE₂ wurde bei dem Haut-Modell nach einmaliger Inkubation für 24 Stunden und nach einer anschließenden zweiten Inkubation für 24 Stunden Inkubation mit Testsubstanzen in den Medienüberständen der Äquivalente quantitativ durch ELISA ermittelt. Als Testsubstanzen wurden die Proben 186-355 sowie unterschiedliche Konzentrationen von SDS verwendet.

### Synthese von PGE₂ nach SDS-Stimulation

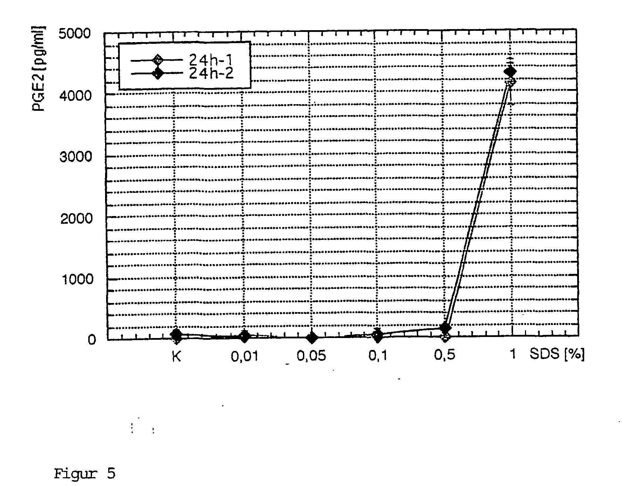

Die Synthese von PGE₂ bleibt im Haut-Modell bis zu einer SDS Konzentration von 0,5% nahezu unverändert gering und steigt aber bereits nach den ersten 24 Stunden bei einer Konzentration von 1% SDS steil an bis auf mehr als 4000 pg/ml pro Hautäquivalent. Die Werte sind nach der zweiten Inkubation für 24 Stunden praktisch unverändert (Figur 5).

### Synthese von PGE₂ nach Inkubation mit den Proben

Im Haut-Modell wurde durch die Proben eine deutliche PGE₂ Synthese induziert.

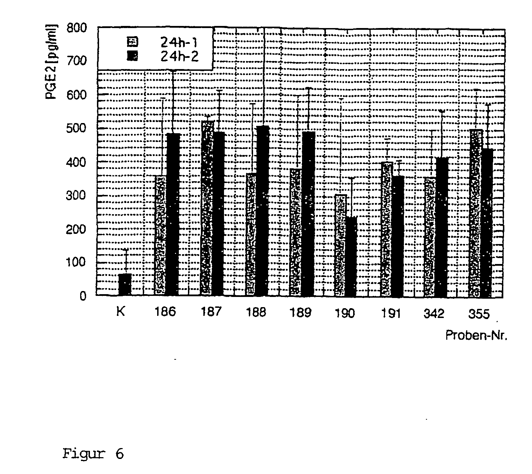

Figur 5 zeigt den Einfluss der Referenzsubstanz SDS auf die Synthese von PGE₂ in den erfindungsgemäßen Hautäquivalenten, während Figur 6 den Einfluss der getesteten Proben auf die PGE₂-Synthese zeigt.

Zusammenfassend kann festgestellt werden, dass das Haut-Modell sehr sensibel und differenziert auf Irritationen reagiert. So ist beispielsweise bei der I11α-Ausschüttung durch SDS ein konzentrationsabhängiger Anstieg zu beobachten. Die untersuchten Proben zeigten ebenfalls eine deutlich verstärkte I11α-Sekretion mit zunehmender Inkubationszeit.

Die Untersuchung der PGE₂-Synthese zeigt, dass beim Haut-Modell durch Irritation eine vermehrte Ausschüttung ausgelöst wird. So steigt die PGE₂-Synthese stark an. Die Werte sind vergleichbar mit der Irritationsschwelle von SDS Ec50 von 0,73%. Deutliche Werte konnten auch nach Inkubation der Proben gemessen werden.

### d) Histologische Veränderungen der Hautäquivalente

Der morphologische Aufbau aller getesteten Äquivalente wurde im Anschluss an die EZ4U-Versuche histologisch untersucht und ausgewertet. Die histologischen Schnitte zeigten eine differenzierte Schädigung in Abhängigkeit des Irritationsgrades. Die untersuchten Proben haben nach zweimal 24 Stunden Inkubation mit mehrmaligem Auftragen der Probe die Verhornung teilweise aufgeweicht, die proliferativen Zellschichten aufgelockert und mehr oder weniger geschädigt.

## Patentansprüche

1. Verfahren zur Vermehrung von dermalen Fibroblasten, wobei die dermalen Fibroblasten eingebettet in einer dreidimensionalen, gelartigen Biomatrix, enthaltend 3,5 bis 4,5 mg/ml Kollagen in gepuffertem serumhaltigen Zellkulturmedium, wobei die für die Herstellung der Biomatrix verwendete Kollagenlösung einen Anteil von ≥ 90 % an nicht-denaturiertem, nativem Kollagen enthält, kultiviert werden.

2. Verfahren zur Überprüfung der Funktion, Morphologie und/oder des Differenzierungsstatus' von dermalen Fibroblasten, wobei die zu überprüfenden dermalen Fibroblasten in eine dreidimensionale, gelartige Biomatrix, enthaltend 3,5 bis 4,5 mg/ml Kollagen in gepuffertem serum-haltigen Zellkulturmedium, wobei die für die Herstellung der Biomatrix verwendete Kollagenlösung einen Anteil von ≥ 90 % an nicht-denaturiertem, nativem Kollagen enthält, eingebracht, in dieser kultiviert und gleichzeitig oder danach überprüft werden.

3. Verfahren zur Herstellung eines in vitro-Dermisäquivalentes, wobei dermale Fibroblasten in eine dreidimensionale, gelartige Biomatrix, enthaltend 3,5 bis 4,5 mg/ml Kollagen in gepuffertem serum-haltigen Zellkulturmedium, wobei die für die Herstellung der Biomatrix verwendete Kollagenlösung einen Anteil von ≥ 90 % an nicht-denaturiertem, nativem Kollagen enthält, eingebettet und in dieser so kultiviert werden, dass ein in vitro-Dermisäquivalent gewonnen wird.

4. Verfahren nach Anspruch 3, wobei die Kultivierung der dermalen Fibroblasten eine mindestens ein- bis zweitägige Submers-Kultur umfasst.

5. Verfahren zur Herstellung eines in vitro-Dermisäquivalentes nach Anspruch 3 oder 4, umfassend das Isolieren von kollagenhaltigem Gewebe, das Überführen des kollagenhaltigen Gewebes in saure Lösung, das Inkubieren des in die saure Lösung überführten Kollagengewebe bei 2 bis 10°C, insbesondere 4°C, das Abzentrifugieren nicht gelöster Kollagenanteile, das Mischen der erhaltenen Kollagenlösung bei 2 bis 10°C, vorzugsweise 4°C, mit einer Lösung, enthaltend die dermalen Fibroblasten, Zellkulturmedium, Serum und Puffer, und das Gelieren der gemischten Lösung durch Erhöhung der Temperatur.

6. Verfahren nach einem der Ansprüche 3 bis 5, wobei die dermalen Fibroblasten nach Kultivierung in der dreidimensionalen, gelartigen Biomatrix aus dieser herausgelöst und in höherer Zelldichte weiter kultiviert werden, so dass ein in vitro-Dermisäquivalent gewonnen wird.

7. Verfahren zur Herstellung eines dreidimensionalen in vitro-Hautäquivalentes, wobei dermale Fibroblasten in eine dreidimensionale, gelartige Biomatrix, enthaltend 3,5 bis 4,5 mg/ml Kollagen in gepuffertem serum-haltigen Zellkulturmedium, wobei die für die Herstellung der Biomatrix verwendete Kollagenlösung einen Anteil von ≥ 90 % an nicht-denaturiertem, nativem Kollagen enthält, eingebettet und in dieser einer mindestens ein- bis zweitägigen Submers-Kultur unterworfen werden und wobei danach Keratinocyten in einem Zellkulturmedium auf der Biomatrix ausgesät und danach weiter kultiviert werden, so dass ein dreidimensionales in vitro-Hautäquivalent gewonnen wird.

8. Verfahren nach Anspruch 7, wobei die Kultivierung der Keratinocyten mindestens eine 5- bis 6-tägige Submers-Kultur und mindestens eine 12-bis 14-tägige Airlift-Kultur umfasst.

9. Verfahren nach Anspruch 7 oder 8, wobei die Keratinocyten einen hohen Anteil undifferenzierter basaler Stammzellen aufweisen.

10. Verfahren nach einem der Ansprüche 7 bis 9, wobei vor, während oder nach der Aussaat der Keratinocyten andere Hautzelltypen, wie Melanocyten, Immunzellen und/oder Endothelzellen, auf der Biomatrix ausgesät und kultiviert werden.

11. Verfahren zur Herstellung eines dreidimensionalen in vitro-Hautäquivalentes nach einem der Ansprüche 7 bis 10, umfassend das Isolieren von kollagenhaltigem Gewebe, das Überführen des kollagenhaltigen Gewebes in saure Lösung, das Inkubieren des in die saure Lösung überführten Kollagengewebe bei 2 bis 10°C, insbesondere 4°C, das Abzentrifugieren nicht gelöster Kollagenanteile, das Mischen der erhaltenen Kollagenlösung bei 2 bis 10°C, vorzugsweise 4°C, mit einer Lösung, enthaltend die dermalen Fibroblasten, Zellkulturmedium, Serum und Puffer, das Gelieren der gemischten Lösung durch Erhöhung der Temperatur, das Inkubieren des gelierten Gemisches bei 37°C und das Aussäen der Keratinocyten und/oder der anderen Hautzelltypen auf das inkubierte, gelierte Gemisch.

12. Verfahren nach einem der Ansprüche 1 bis 11, wobei die eingebettete dermale Fibroblasten enthaltende Biomatrix hergestellt wird, indem aus einem Gewebe isolierte Kollagenfasern in saurer Lösung 3 bis 14 Tage bei 2 bis 10°C, vorzugsweise 4°C, gerührt, nicht gelöste Kollagenanteile abzentrifugiert und die erhaltene fertige Kollagenlösung mit einem Kollagengehalt von 3 mg/ml bis 8 mg/ml mit einer Lösung, enthaltend dermale Fibroblasten, Zellkulturmedium, Serum und Puffer, bei 2 bis 10°C, vorzugsweise 4°C, gemischt und anschließend bei höherer Temperatur, vorzugsweise bei Raumtemperatur bis 37°C, geliert wird.

13. Verfahren nach Anspruch 12, wobei die saure Lösung Essigsäurelösung, insbesondere 0,1 %-ige Essigsäurelösung, ist.

14. Verfahren nach Anspruch 12 oder 13, wobei die Lösung, enthaltend Zellkulturmedium, Serum und Puffer, im 1:2-Verhältnis mit der kollagenhaltigen Lösung gemischt wird.

15. In vitro-Dermisäquivalent, hergestellt nach einem Verfahren gemäß einem der Ansprüche 3 bis 6 oder 12 bis 14.

16. Humanes in vitro-Dermisäquivalent gemäß Anspruch 15, hergestellt unter Verwendung humaner dermaler Fibroblasten.

17. Dreidimensionales in vitro-Hautäquivalent, hergestellt nach einem Verfahren gemäß einem der Ansprüche 7 bis 14.

18. Humanes dreidimensionales in vitro-Hautäquivalent gemäß Anspruch 17, hergestellt unter Verwendung humaner dermaler Fibroblasten und humaner Keratinocyten und gegebenenfalls anderer humaner Hautzelltypen.

19. Verfahren zur Bestimmung der Wirkung einer chemischen Substanz oder eines Agens auf Dermiszellen, umfassend das Inkontaktbringen der chemischen Substanz oder des Agens mit einem in vitro-Dermisäquivalent gemäß Anspruch 15 oder 16 und Bestimmung der Wechselwirkung zwischen Dermisäquivalent und der chemischen Substanz oder dem Agens.

20. Verfahren zur Bestimmung der Wirkung einer chemischen Substanz oder eines Agens auf Hautzellen, umfassend das Inkontaktbringen der chemischen Substanz oder des Agens mit einem dreidimensionalen in vitro-Hautäquivalent gemäß Anspruch 17 oder 18 und Bestimmung der Wechselwirkung zwischen Hautäquivalent und der chemischen Substanz oder dem Agens.

## Claims

1. Method for multiplying dermal fibroblasts, wherein the dermal fibroblasts are cultivated embedded in a three-dimensional, gel-like biomatrix containing 3.5 to 4.5 mg/ml collagen in buffered serum-containing cell culture medium, the collagen solution which is used for producing the biomatrix containing a proportion of ≥ 90% of non-denatured, native collagen.

2. Method for examining the function, morphology and/or the differentiation status of dermal fibroblasts, wherein the dermal fibroblasts which are to be examined are introduced into a three-dimensional, gel-like biomatrix containing 3.5 to 4.5 mg/ml collagen in buffered serum-containing cell culture medium, the collagen solution which is used for producing the biomatrix containing a proportion of ≥ 90% of non-denatured, native collagen, are cultivated in said biomatrix and are examined at the same time or thereafter.

3. Method for producing an in vitro dermis equivalent, wherein dermal fibroblasts are embedded in a three-dimensional, gel-like biomatrix containing 3.5 to 4.5 mg/ml collagen in buffered serum-containing cell culture medium, the collagen solution which is used for producing the biomatrix containing a proportion of ≥ 90% of non-denatured, native collagen, and are cultivated in said biomatrix such that an in vitro dermis equivalent is obtained.

4. Method according to claim 3, wherein the cultivation of the dermal fibroblasts comprises an at least one- to two-day submerged culture.

5. Method for producing an in vitro dermis equivalent according to claim 3 or 4, comprising isolating collagen-containing tissue, transferring the collagen-containing tissue into acid solution, incubating the collagen tissue which has been transferred into the acid solution at 2 to 10°C, in particular 4°C, centrifuging-off non-dissolved collagen components, mixing the obtained collagen solution at 2 to 10°C, preferably 4°C, with a solution containing the dermal fibroblasts, cell culture medium, serum and buffer, and gelling the mixed solution by raising the temperature.

6. Method according to one of the claims 3 to 5, wherein the dermal fibroblasts, after cultivation in the three-dimensional gel-like biomatrix, are dissolved out of the latter and are cultivated further at a higher cell density, so that an in vitro dermis equivalent is obtained.

7. Method for producing a three-dimensional, in vitro skin equivalent, wherein dermal fibroblasts are embedded in a three-dimensional, gel-like biomatrix containing 3.5 to 4.5 mg/ml collagen in buffered serum-containing cell culture medium, the collagen solution which is used for producing the biomatrix containing a proportion of ≥ 90% of non-denatured, native collagen, and are subjected in said biomatrix to an at least one- to two-day submerged culture and wherein thereafter keratinocytes in a cell culture medium are seeded on the biomatrix and thereafter are cultivated further, so that a three-dimensional, in vitro skin equivalent is obtained.

8. Method according to claim 7, wherein the cultivation of the keratinocytes comprises at least a 5- to 6-day submerged culture and at least a 12- to 14-day airlift culture.

9. Method according to claim 7 or 8, wherein the keratinocytes have a high proportion of undifferentiated basal stem cells.

10. Method according to one of the claims 7 to 9, wherein other skin cell types, such as melanocytes, immune cells and/or endothelial cells, are seeded on the biomatrix and are cultivated before, during or after the seeding of the keratinocytes.

11. Method for producing a three-dimensional, in vitro skin equivalent according to one of the claims 7 to 10, comprising isolating collagen-containing tissue, transferring the collagen-containing tissue into acid solution, incubating the collagen tissue which has been transferred into the acid solution at 2 to 10°C, in particular 4°C, centrifuging-off non-dissolved collagen components, mixing the obtained collagen solution at 2 to 10°C, preferably 4°C, with a solution containing the dermal fibroblasts, cell culture medium, serum and buffer, gelling the mixed solution by raising the temperature, incubating the gelled mixture at 37°C and seeding the keratinocytes and/or the other skin cell types on the incubated, gelled mixture.

12. Method according to one of the claims 1 to 11, wherein the biomatrix containing embedded dermal fibroblasts is produced in that collagen fibres which have been isolated from a tissue are agitated in acid solution for 3 to 14 days at 2 to 10°C, preferably 4°C, non-dissolved collagen components are centrifuged-off and the obtained prepared collagen solution with a collagen content of 3 mg/ml to 8 mg/ml is mixed with a solution containing dermal fibroblasts, cell culture medium, serum and buffer at 2 to 10°C, preferably 4°C, and subsequently is gelled at a higher temperature, preferably at room temperature to 37°C.

13. Method according to claim 12, wherein the acid solution is acetic acid solution, in particular 0.1% acetic acid solution.

14. Method according to claim 12 or 13, wherein the solution containing cell culture medium, serum and buffer is mixed with the collagen-containing solution in the ratio 1:2.

15. In vitro dermis equivalent, produced according to a method according to one of the claims 3 to 6 or 12 to 14.

16. Human, in vitro dermis equivalent according to claim 15, produced using human dermal fibroblasts.

17. Three-dimensional, in vitro skin equivalent, produced according to a method according to one of the claims 7 to 14.

18. Human, three-dimensional, in vitro skin equivalent according to claim 17, produced using human dermal fibroblasts and human keratinocytes and if necessary other human skin cell types.

19. Method for determining the effect of a chemical substance or of an agent on dermis cells, comprising bringing the chemical substance or the agent into contact with an in vitro dermis equivalent according to claim 15 or 16 and determining the interaction between dermis equivalent and the chemical substance or the agent.

20. Method for determining the effect of a chemical substance or of an agent on skin cells, comprising bringing the chemical substance or the agent into contact with a three-dimensional, in vitro skin equivalent according to claim 17 or 18 and determining the interaction between skin equivalent and the chemical substance or the agent.

## Revendications

1. Procédé de multiplication des fibroblastes dermiques, selon lequel on cultive les fibroblastes dermiques enrobés dans une biomatrice tridimensionnelle gélatineuse contenant de 3,5 à 4,5 mg/ml de collagène dans un milieu de culture cellulaire contenant du sérum tamponné, la solution de collagène utilisée pour la production de la biomatrice contenant une proportion ≥ 90 % de collagène natif non dénaturé.

2. Procédé de vérification de la fonction, de la morphologie et/ou de l'état de différentiation de fibroblastes dermiques, selon lequel on introduit les fibroblastes dermiques à tester dans une biomatrice tridimensionnelle gélatineuse contenant de 3,5 à 4,5 mg/ml de collagène dans un milieu de culture cellulaire contenant du sérum tamponné, la solution de collagène utilisée pour la production de la biomatrice contenant une proportion ≥ 90 % de collagène natif non dénaturé, on les y cultive et simultanément ou ensuite on les teste.

3. Procédé de production d'un modèle de peau in vitro, selon lequel on enrobe des fibroblastes dermiques dans une matrice tridimensionnelle gélatineuse contenant de 3,5 à 4,5 mg/ml de collagène dans un milieu de culture cellulaire contenant du sérum tamponné, la solution de collagène utilisée pour la production de la biomatrice contenant une proportion ≥ 90 % de collagène natif non dénaturé et on les y cultive de manière à obtenir un modèle de peau in vitro.

4. Procédé selon la revendication 3,
**caractérisé en ce que**
la culture des fibroblastes dermiques comprend une culture immergée pendant au moins un à deux jours.

5. Procédé de production d'un modèle de peau in vitro selon la revendication 3 ou 4, comprenant l'isolement de tissu contenant du collagène, le transfert de tissu contenant du collagène en solution acide, l'incubation du tissu de collagène transféré dans la solution acide entre 2 et 10°C, en particulier 4°C, la centrifugation des parties de collagène non dissoutes, le mélange de la solution de collagène obtenue entre 2 et 10°C, de préférence à 4°C, avec une solution contenant les fibroblastes dermiques, le milieu de culture cellulaire, du sérum et un tampon, et la gélification de la solution obtenue par élévation de la température.

6. Procédé selon une des revendications 3 à 5,
**caractérisé en ce qu'**
on extrait par un solvant les fibroblastes dermiques après culture dans la biomatrice tridimensionnelle gélatineuse et on en poursuit la culture dans une densité cellulaire supérieure, de manière à obtenir un modèle de peau in vitro.

7. Procédé de production d'un modèle de peau tridimensionnel in vitro,
**caractérisé en ce qu'**
on enrobe des fibroblastes dermiques dans une biomatrice tridimensionnelle gélatineuse contenant de 3,5 à 4,5 mg/ml de collagène dans un milieu de culture cellulaire contenant du sérum tamponné, la solution de collagène utilisée pour la production de la biomatrice contenant une proportion ≥ 90 % de collagène natif non dénaturé, et on les y soumet à une culture en immersion pendant au moins un à deux jours, et ensuite on ensemence des kératinocytes dans un milieu de culture cellulaire sur la biomatrice puis on les cultive encore de manière à obtenir un modèle de peau tridimensionnel in vitro.

8. Procédé selon la revendication 7,
**caractérisé en ce que**
la culture des kératinocytes comprend au moins une culture en immersion pendant 5 à 6 jours et au moins une culture avec agitation par circulation d'air pendant 12 à 14 jours.

9. Procédé selon la revendication 7 ou 8,
**caractérisé en ce que**
les kératinocytes présentent une proportion élevée de cellules souches basales indifférenciées.

10. Procédé selon l'une des revendications 7 à 9,
**caractérisé en ce qu'**
avant, pendant ou après l'ensemencement des kératinocytes, on sème sur la biomatrice et on cultive d'autres types de cellules dermiques, comme des mélanocytes, des cellules immunitaires et/ou des cellules endothéliales.

11. Procédé de production d'un modèle de peau tridimensionnel in vitro selon l'une des revendications 7 à 10, comprenant l'isolement de tissu contenant du collagène, le transfert du tissu contenant du collagène en solution acide, l'incubation du tissu de collagène transféré dans la solution acide entre 2 et 10°C, en particulier 4°C, la centrifugation des parties de collagène non dissoutes, le mélange de la solution de collagène obtenue entre 2 et 10°C, de préférence à 4°C, avec une solution contenant les fibroblastes dermiques, le milieu de culture cellulaire, du sérum et un tampon, la gélification de la solution mélangée par élévation de la température, l'incubation du mélange gélifié à 37°C et l'ensemencement des kératinocytes et/ou des autres types de cellules dermiques sur le mélange gélifié incubé.

12. Procédé selon l'une des revendications 1 à 11,
**caractérisé en ce qu'**
on produit la biomatrice contenant les fibroblastes dermiques enrobés en agitant les fibres de collagène isolées à partir d'un tissu en solution acide pendant 3 à 14 jours entre 2 et 10°C , de préférence 4°C, en séparant par centrifugation les parties de collagène non dissoutes et en mélangeant la solution de collagène prête à l'emploi obtenue, ayant une teneur en collagène de 3 mg/ml à 8 mg/ml, avec une solution contenant des fibroblastes dermiques, un milieu de culture cellulaire, du sérum et un tampon, entre 2 et 10°C, de préférence à 4°C, puis en gélifiant à température plus élevée, de préférence entre la température ambiante et 37°C.

13. Procédé selon la revendication 12,
**caractérisé en ce que**
la solution acide est une solution d'acide acétique, en particulier une solution d'acide acétique à 0,1 %.

14. Procédé selon la revendication 12 ou 13,
**caractérisé en ce qu'**
on mélange la solution contenant le milieu de culture cellulaire, du sérum et un tampon, dans un rapport 1 : 2 avec la solution contenant du collagène.

15. Modèle de peau in vitro produit selon un procédé de l'une des revendications 3 à 6 ou 12 à 14.

16. Modèle de peau humaine in vitro selon la revendication 15, produit en utilisant des fibroblastes dermiques humains.

17. Modèle de peau tridimensionnel in vitro, produit selon un procédé de l'une des revendications 7 à 14.

18. Modèle de peau humaine tridimensionnel in vitro selon la revendication 17, produit en utilisant des fibroblastes dermiques humains et des kératinocytes humains, ainsi que le cas échéant d'autres types de cellules dermiques humaines.

19. Procédé de détermination de l'action d'une substance chimique ou d'un agent sur des cellules dermiques, comprenant la mise en contact de la substance chimique ou de l'agent avec un modèle de peau in vitro selon la revendication 15 ou 16, et la détermination de l'interaction entre le modèle de peau et la substance chimique ou l'agent.

20. Procédé de détermination de l'action d'une substance chimique ou d'un agent sur des cellules dermiques, comprenant la mise en contact de la substance chimique ou de l'agent avec un modèle de peau in vitro selon la revendication 17 ou 18, et la détermination de l'interaction entre le modèle de peau et la substance chimique ou l'agent.
